# EUROPEAN PATENT SPECIFICATION

(11) **EP 3 595 628 B1**
(45) Date of publication and mention of the grant of the patent: **01.12.2021**
(21) Application number: 18712178.5
(22) Date of filing: 16.03.2018
(51) Int. Cl.: A61K 8/49, A61Q 19/00, A61K 47/08, A61K 47/20, A61K 9/06, A61K 9/08, A61K 31/4045, A61K 47/10

(54) **COMPOSITIONS FOR RETARDING THE DECOMPOSITION OF MELATONIN IN SOLUTION**
ZUSAMMENSETZUNGEN ZUR VERZÖGERUNG DER ZERSETZUNG VON MELATONIN IN LÖSUNGEN
COMPOSITIONS POUR RETARDER LA DÉCOMPOSITION DE LA MÉLATONINE EN SOLUTION

(30) Priority: 16.03.2017 DK PA201770185
(43) Date of publication of application: 22.01.2020
(73) Proprietor: RepoCeuticals A/S, 2970 Hørsholm (DK)
(72) Inventor: UTTENTHAL, Lars Otto, 28009 Madrid (ES); LINDBLAD, Lasse, 3140 Ålsgårde (DK)
(74) Representative: Zacco Denmark A/S
(86) International application number: PCT/EP2018/056670
(87) International publication number: WO 2018/167273

(56) References cited:
- WO-A1-2008/127609
- US-A1- 2010 197 758
- US-A1- 2013 267 489
- US-A1- 2015 250 884

## Description

### Field of the invention

The present invention provides compositions comprising biocompatible substances that diminish the breakdown of melatonin in aqueous solutions and is hence of use in the manufacture and storage of medicinal products or food supplements comprising melatonin in such solutions or liquid or semi-liquid aqueous preparations in order to prolong their shelf life with adequate preservation of melatonin bioactivity. As such, it is relevant to the pharmaceutical, natural medicine and/or food supplement industries.

### Background of the invention

**Melatonin:** Melatonin (*N*-acetyl-5-methoxytryptamine) is a hormone produced by the pineal gland in human beings and other mammals by enzymatic modification of the amino acid tryptophan. Melatonin is involved in maintaining the circadian rhythm of various biological functions, being secreted in hours of darkness and acting on high-affinity melatonin Gᵢ-coupled transmembrane receptors MT1 and MT2, which are widely distributed in many cells and tissues of the body. At the same time melatonin acts at supraphysiological concentrations as a powerful antioxidant and free radical scavenger for hydroxyl ions and other reactive oxygen species (ROS) as well as reactive nitrogen species. Because of its efficiency as a free radical scavenger, melatonin has been proposed as an agent to protect cells and tissues against injury from ionizing radiation. Melatonin can also activate cytoprotective antioxidative enzymes such as copper-zinc and manganese superoxide dismutases (CuZnSOD and MnSOD) and glutathione peroxidase. In addition, melatonin has anti-inflammatory effects to prevent the upregulation or cause the down-regulation of the expression of nuclear factor kappa B (NF-kB) and pro-inflammatory cytokines such as tumor necrosis factor alpha (TNF-α) and interleukin 1 beta (IL-1β).

**Melatonin in solution or semi-liquid preparations:** The commonest medicinal or quasi-medicinal use of melatonin is as an agent to aid falling asleep at bedtime or to reset the diurnal sleep/wakefulness cycle when this has been disturbed by travel across time-zones (jet lag) or shift work. The commonest mode of administration is to take solid melatonin orally in tablet or capsule form, but melatonin can also be given sublingually in the form of a solution applied as drops or in a spray. Melatonin has also been used locally as solutions, lotions, creams or ointments to be applied to the skin. This may be done with the implied intention of using the transdermal absorption of melatonin to promote sleep, or of using the topical melatonin as a prophylactic or therapeutic remedy for radiation dermatitis or sunburn. Information on the stability of melatonin in such commercially available liquid or semi-liquid products is not usually publicly available.

Published studies on the stability of melatonin in aqueous solutions have given conflicting results. Lee (1990) found substantial rates of degradation of melatonin at concentrations of about 4 µg/mL in aqueous buffer solutions at pH 3.3, 6.1 and 8.7, containing 10%, 20%, 40% and 80% propylene glycol at 25°C. Melatonin concentrations fell to 39%-81% of the starting concentration within 7 days. In contrast, Cavallo and Hassan (1995) concluded that melatonin at low concentrations (1-113 µg/mL) in aqueous solutions without propylene glycol were stable for up to 6 months in sterile, pyrogen-free vials at 4°C or -70°C. However, Daya et al (2001) found that melatonin solutions of 50 µg/mL in phosphate buffers at pH 1.2, 2.4, 7.4, 10 and 12 were all unstable at 20°C and 37°C, melatonin concentrations declining to 70%-78% of the starting concentration in 21 days. The decline was lowest at acid pH, but was not accelerated at the higher temperature. While the data are conflicting, they imply that aqueous melatonin solutions will not generally be sufficiently stable at room temperature to permit an adequate shelf life for use as medicinal products unless the solutions contain additives that inhibit the breakdown of melatonin. The evidence suggests that propylene glycol will not be such an additive and that it might even have a deleterious effect on melatonin stability.

**Need for stable solutions of melatonin for topical application:** Particularly in connection with the topical application of melatonin preparations for protecting the skin or other epithelia from deleterious effects of ionizing radiation, there is a need for developing solutions or other liquid and semi-liquid preparations of melatonin in which the melatonin is present in sufficient concentration to exert its protective effect and is also protected from rapid breakdown, such that the preparations will have an adequate shelf life for convenient storage and use. In particular in regions of our planet, where local temperatures are elevated and low temperature storage facilities for medical compositions are sparse, there is a need for compositions comprising melatonin and derivatives thereof for topical applications against a variety of diseases of e.g. the skin, which remain stable at elevated temperatures for long periods of time after preparation.

### Summary of the invention

We have now surprisingly found that melatonin dissolved in a glycofurol/water mixture is not subject to the rapid degradation that has been seen in propylene glycol/water mixtures, but that the minor degree of degradation that does occur in the mixture is prevented by the presence of dimethyl sulfoxide (DMSO) in the mixture. Accordingly, the invention comprises elements and aspects as detailed herein.

In one aspect, the present invention relates to the use of DMSO as an additive in compositions of melatonin as set out in the appended claims in order to prolong the shelf life of the composition by retarding the breakdown of the active ingredient.

In one embodiment thereof the active ingredient is dissolved in a glycofurol/DMSO/water mixture.

The compositions of the invention are used for topical application to the skin and other epithelia, including skin wounds or ulcers, the oropharyngeal cavity, the rectum, vagina, or the urothelium of the urinary bladder.

In particular, the present invention relates to the following aspects and embodiments:
In a first aspect there is disclosed the use of DMSO as an additive in a solution comprising a solvent and at least one active ingredient selected from melatonin and/or an antioxidant metabolite, derivative or analogue thereof, for prolonging the stability and/or shelf life of the at least one active ingredient in the solution by retarding a decomposition of the at least one active ingredient comprised in the solution.

In an embodiment of the first aspect, the use of DMSO, wherein the active ingredient is present in the solution to at least 95% of initial amount after 45 days of storage at 25°C.

In an embodiment of the first aspect, the use of DMSO, wherein the solvent is water.

In an embodiment of the first aspect, the use of DMSO, wherein the solution further comprises a water-miscible and biocompatible solvent or solubilizer of the at least one active ingredient.

In an embodiment of the first aspect, the use of DMSO, wherein the water-miscible and biocompatible solvent or solubilizer is glycofurol.

In an embodiment of the first aspect, the use of DMSO, wherein DMSO is present in a molar concentration at least 2-fold higher than the molar concentration of the at least one active ingredient.

In an embodiment of the first aspect, the use of DMSO, wherein the solution is an aqueous solution comprising between 20% (w/w) and 60% (w/w) of DMSO based on the total mass of DMSO and water.

In an embodiment of the first aspect, the use of DMSO, wherein the solution comprises 40% (w/w) or 50% (w/w) of DMSO based on the total mass of DMSO and water.

In an embodiment of the first aspect, the use of DMSO, wherein the solution is an aqueous solution comprising between 10% (w/w) and 30% (w/w) of glycofurol based on the total mass of glycofurol and water; preferably 20% (w/w) glycofurol.

In an embodiment of the first aspect, the use of DMSO, wherein the solution is an aqueous solution comprising 20% (w/w) of glycofurol based on the total mass of glycofurol and water, and 40% (w/w) of DMSO based on the total mass of DMSO and water.

Further, there is disclosed in a second aspect and first embodiment, the use of glycofurol as an additive in a solution comprising a solvent and at least one active ingredient selected from melatonin and/or an antioxidant metabolite, derivative or analogue thereof, for prolonging stability and/or shelf life of the at least one active ingredient in the solution by retarding a decomposition of the at least one active ingredient comprised in the solution.

In an embodiment of the second aspect, the use of glycofurol, wherein the active ingredient is present in the solution to at least 95% of initial amount after 45 days of storage at 25°C.

In an embodiment of the second aspect, the use of glycofurol, wherein the solvent is water.

In an embodiment of the second aspect, the use of glycofurol, wherein the solution is an aqueous solution comprising between 10% (w/w) and 30% (w/w) of glycofurol based on the total mass of glycofurol and water; preferably 20% (w/w) of glycofurol.

In an embodiment of the second aspect, the use of glycofurol, wherein the solution further comprises DMSO.

In an embodiment of the second aspect, the use of glycofurol, wherein DMSO is present in a molar concentration at least 2-fold higher than the molar concentration of the at least one active ingredient.

In an embodiment of the second aspect, the use of glycofurol, wherein the solution is an aqueous solution comprising between 20% (w/w) and 60% (w/w) of DMSO based on the total mass of DMSO and water.

In an embodiment of the second aspect, the use of glycofurol, wherein the aqueous solution comprises 40% (w/w) or 50% (w/w) of DMSO based on the total mass of DMSO and water.

In an embodiment of the second aspect, the use of glycofurol, wherein the solution is an aqueous solution comprising 20% (w/w) of glycofurol based on the total mass of glycofurol and water; and 40% (w/w) of DMSO based on the total mass of DMSO and water.

Further, there is disclosed in a third aspect, a solution comprising a solvent, at least one active ingredient selected from melatonin and/or an antioxidant metabolite, derivative or analogue thereof, and either DMSO, or a water-miscible and biocompatible solvent or solubilizer of the at least one active ingredient, or both DMSO and a water-miscible and biocompatible solvent or solubilizer of the at least one active ingredient.

In an embodiment of the third aspect, a solution wherein the active ingredient is present in the solution to at least 95% of initial after 45 days of storage at 25°C.

In an embodiment of the third aspect, a solution wherein the solvent is water.

In an embodiment of the third aspect, a solution wherein the water-miscible and biocompatible solvent or solubilizer of the at least one active ingredient is glycofurol.

In an embodiment of the third aspect, a solution wherein DMSO, when present, is present in a molar concentration at least 2-fold higher than the molar concentration of the at least one active ingredient.

In an embodiment of the third aspect, a solution wherein the solution is an aqueous solution comprising between 20% (w/w) and 60% (w/w) of DMSO based on the total mass of DMSO and water.

In an embodiment of the third aspect, a solution wherein the solution comprises 40% (w/w) or 50% (w/w) of DMSO based on the total mass of DMSO and water.

In an embodiment of the third aspect, a solution wherein the solution is an aqueous solution comprising between 10% (w/w) and 30% (w/w) of glycofurol based on the total mass of glycofurol and water; preferably 20% (w/w) of glycofurol.

In an embodiment of the third aspect, a solution wherein the solution is an aqueous solution comprising 20% (w/w) of glycofurol based on the total mass of glycofurol and water, and 40% (w/w) of DMSO based on the total mass of DMSO and water.

Further in a fourth aspect there is disclosed, a composition comprising a solution according to any embodiment of the third aspect for topical application to a human for treatment of a medical condition of the human responsive to an active ingredient comprised in the composition, wherein the medical condition comprises a medical condition of the skin and other epithelia of the human, such as a non-healing skin wound, an ulcer, the oropharyngeal cavity, the rectum, the vagina, or the urothelium of the urinary bladder; therein characterized by the active ingredient being present in the composition after 45 days of storage at 25°C to at least 95% of initial amount at preparation of the solution.

### Brief description of the drawings

Figure 1 details experimental results from HPLC of melatonin 10 mg/mL in 20% (w/w) aqueous glycofurol previously stored for 45 days at 5°C (Figure 1A) or 25°C (Figure 1B).
Figure 2 details experimental results from HPLC of melatonin 10 mg/mL in 20% (w/w) aqueous glycofurol and 40% (w/w) aqueous DMSO previously stored for 45 days at 5°C (Figure 2A) or 25°C (Figure 2B).
Figure 3 details experimental results from HPLC of melatonin 10 mg/mL in 50% (w/w) aqueous DMSO previously stored for 45 days at 5°C (Figure 3A) or 25°C (Figure 3B).

### Detailed description of the invention

The present invention provides compositions in which DMSO is used as a preservative to prolong the shelf life and bioactivity of preparations comprising melatonin or an antioxidant metabolite, derivative or analogue thereof as an active ingredient.

**Melatonin:** The essential features of melatonin (molecular weight 232.3 g/mol) as a circadian-rhythm-regulating hormone, anti-oxidant, free-radical scavenger and anti-inflammatory agent have been outlined above. Because of melatonin's efficiency as a free radical scavenger, especially of hydroxyl radicals (Tan et al 1993) and ROS, it has been proposed as an agent to protect against radiation injury to cells and tissues. This has been studied chiefly by giving large systemic doses of melatonin to rodents which are then subjected to large doses of whole-body irradiation. However, there have also been studies on the action of topically applied melatonin to prevent or alleviate sun scalding. Melatonin has been demonstrated to protect against the adverse effects of all relevant wavelengths of ionizing radiation from ultraviolet through x-rays to gamma rays. The results of such studies have been reviewed by Vijayalaxmi et al (2004).

**Melatonin metabolites, derivatives and analogues:** Many chemical derivatives of melatonin, including breakdown products and natural metabolites of melatonin, retain the antioxidant and free-radical scavenging properties of the parent molecule. This makes melatonin a more effective antioxidant than other natural antioxidants such as vitamins C and E (cited by Reiter et al 2007). However, these vitamins show synergy with melatonin with respect to antioxidant activity. In non-hepatic tissues, the reaction of melatonin with two hydroxyl radicals yields the metabolite cyclic 3-hydroxymelatonin (C3-OHM), which undergoes further oxidation by two hydroxyl radicals to break the indole ring and form *N*¹-acetyl-*N*²-formyl-5-methoxykynuramine (AFMK) (Tan et al 1993; Reiter et al 2007). C3-OHM is therefore also an effective antioxidant and hydroxyl radical scavenger. The reaction of melatonin with the hydroxyl radical precursor, hydrogen peroxide, similarly leads to production of AFMK. AFMK is also a reducing agent, capable of donating electrons to detoxify radical species, and has been shown to preserve the integrity DNA exposed to oxidizing agents. The action of aryl formamidase or catalase on AFMK produces *N*¹-acetyl-5-methoxykynuramine (AMK), which is an even more effective scavenger of hydroxyl radicals and reactive nitrogen species, protecting proteins from oxidative destruction. In this process, 3-acetamidomethyl-6-methoxycinnolinone (AMMC) or 3-nitro-AMK (AMNK) is formed.

The liver is the principal site of the classically reported metabolic pathway for melatonin. This consists chiefly of 6-hydroxylation by the cytochromes P450 CYP1A1, CYP1A2, and CYP1B1, and the formation of the minor metabolite N-acetylserotonin by CYP2C19. The main product 6-hydroxymelatonin (6-OHM) is then conjugated at the hydroxyl group to form the 6-OHM glucuronide or 6-OHM sulfate. 6-OHM is an effective free radical scavenger in a variety of situations, but is also reported to show pro-oxidant effects in others. Its status as an antioxidant thus remains equivocal (Maharaj et al 2007).

*N*-acetylserotonin (NAS) is not only the immediate biosynthetic precursor but also a minor metabolite of melatonin. Like 6-OHM, it is conjugated to form the glucuronide or sulfate. Its protective effect against oxidative damage in certain model systems is reportedly 5 to 20 times as strong as that of melatonin (Oxenkrug 2005).

Melatonin can also be chemically modified by introducing chemical groups at one or more of any of its constituent atoms susceptible of such modification or by introducing such groups in de novo synthesis of melatonin analogues or derivatives. Such modifications, which do not alter the fundamental indole structure of melatonin, are herein called derivatives. The fundamental indole structure of melatonin can also be modified by substituting other bicyclic aromatic structures. Such modifications are herein called analogues, which may also have different chemical side groups removed, introduced or modified. Many such analogues and derivatives have been prepared, but most of them have not been tested for their antioxidant or free-radical scavenging properties.

**Natural antioxidants that may act in synergy with melatonin:** A large number of natural antioxidant agents that have been used pharmaceutically may potentially act synergically with melatonin. These may have additive antioxidant effects, but only a few have been demonstrated to act synergically. Vitamins C and E have been cited in this context. A related but not identical property, which is less well assessed, is their efficiency as free radical scavengers and in conferring protection against the harmful effects of radiation and cytotoxic medication. Further natural antioxidants that come under consideration as conferring addition protective effect are alpha-lipoic acid and coenzyme Q10 (also known as ubidecarenone). Both are effective as free radical scavengers and their capacity to ameliorate radiation damage has been demonstrated *in vitro* and in animal models in which the substances have usually been given intraperitoneally or by dietary supplementation.

**Antioxidant metabolites of melatonin:** Of those described above, *N*¹-acetyl-*N*²-formyl-5-methoxykynuramine (AFMK), 6-hydroxymelatonin (6-OHM) and *N-*acetylserotonin (NAS) can be used in compositions of the invention. Cyclic 3-hydroxymelatonin (C3-OHM) and *N*¹-acetyl-5-methoxykynuramine (AMK) are unstable and hence unsuitable for use in a pharmaceutical composition.

**Antioxidant melatonin derivatives:** The chemical structure of melatonin can be represented as in Figure (I), in which sites suitable for chemical modification by the substitution of different chemical groups have been indicated by R₁, R₂, R₃, R₄, R₅ and R₆. These numbers do not correspond to the conventional numbering of positions in the indole ring of melatonin.

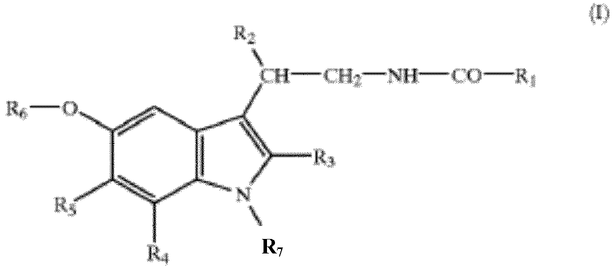

In native melatonin, R₁ and R₆ represent CH₃, while R₂, R₃, R₄, R₅ and R₇ represent H.

Antioxidant melatonin derivatives may comprise, as non-exclusive examples, those in which
R₁ represents H, a linear or branched C₁-C₄ alkyl group or a C₁-C₄ alkoxy group,
R₂ represents H or a C₁-C₄ alkyl group,
R₃ represents H, a methyl group or a halogen atom,
R₄ represents H or a halogen atom,
R₅ represents H or a halogen atom,
R₆ represents H or a linear or branched C₁-C₄ alkyl group,
R₇ represents H, a linear or branched C₁-C₄ alkyl group, a ―C(=O)-O-Rₐ group or a ―C(=O)-N(H)-Rₐ group wherein Rₐ is a linear or branched C₁-C₄ alkyl group, the ―CH₂NH-C(=O)-R₁ side chain is extended by duplicating, triplicating or quadruplicating the ―CH₂- group,
or pharmaceutically acceptable salts of such derivatives.

**Synergically acting antioxidants:** Melatonin or an antioxidant analogue or metabolite thereof may be combined with a synergically acting antioxidant such as vitamin E, coenzyme Q10, alpha-lipoic acid or vitamin C as active substances. These substances may be provided in various forms, analogues or derivatives according to the specific intended use and the required pharmaceutical formulation. For example, vitamin E may be given as the water-soluble D-alpha-tocopheryl succinate and coenzyme Q10 may be substituted by coenzyme Q9, decylubiquinone and idebenone. However, all the above active substances in their non-derivatized forms are readily soluble in DMSO, a biocompatible solvent that has been safely used as a pharmaceutical ingredient in human subjects whether in topical, oral or intravenous preparations.

**Dimethyl sulfoxide (DMSO):** DMSO ((CH₃)₂SO), molecular weight 78.1 g/mol, is a colorless polar aprotic solvent for both polar and nonpolar compounds and is completely miscible with water and a wide range of organic solvents. It is well known as a solvent or solubilizer for melatonin, as are also ethanol, glycerin and propylene glycol. The solubility of melatonin in DMSO at room temperature (20 or 25°C) is quoted by various sources as being up to a concentration of 50 millimolar (mM) (tocris), 100 mM (abcam) or 200 mM (MedChem), equivalent to 11.6, 23.2 or 47 g/L, respectively, but it may in fact be soluble to a 1 molar concentration (232 g/L) or higher. Similar figures are quoted for the solubility of melatonin in ethanol (182 g/L at 20°C; EC SCCS/1315/10, 2010). On the other hand, the solubility of melatonin in water is limited to approximately 2 g/L at 20°C and 5 g/L at 50°C. In an ethanol/water (40/60 v/v) mixture, the solubility of melatonin is 64 g/L at 20°C. DMSO shows low toxicity, the median lethal dose being higher than that of ethanol (DMSO: LD₅₀ oral, rat, 14.5 g/kg; ethanol: LD₅₀ oral, rat, 7.06 g/kg). DMSO penetrates the skin and other epithelia without damaging them and can carry other compounds dissolved in it into the underlying cells and tissues. DMSO has been used in human subjects as a topical analgesic, a vehicle for the topical application of pharmaceuticals e.g. as a component of a transdermal drug delivery systems, as an anti-inflammatory agent, and as an antioxidant. While DMSO has been examined for the treatment of numerous conditions and ailments, its only use approved by the U.S. Food and Drug Administration (FDA) has been for the symptomatic treatment of interstitial cystitis, in which 50% (w/w) aqueous DMSO is instilled into the urinary bladder. DMSO has also been described as having a radioprotective effect, and has itself been used as a free-radical scavenging, antioxidant treatment for radiation cystitis (Shirley et al 1978).

The possibility of DMSO having an influence on the stability of the melatonin in the administered solutions and use of DMSO as an agent to prolong the shelf life of the melatonin solutions seems neither to have been suspected nor studied.

**Glycofurol:** Glycofurol (C₇H₁₄O₃), molecular weight 146.2 g/mol, like DMSO, is regarded as a solvent, solubilizer or penetration agent for compounds that are sparingly soluble in aqueous media.

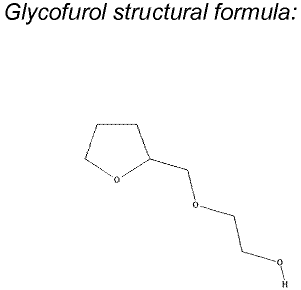

It is used as a solvent in parenteral products for intravenous or intramuscular injection in concentrations up to 50% v/v. It has also been used as a penetration enhancer and solvent in topical and intranasal formulations, and at 20% v/v concentration in a rectal formulation. It is a clear, colorless, almost odorless liquid with a bitter taste, producing a warm sensation on the tongue. It is miscible in all proportions with ethanol, propan-2-ol, propylene glycol, glycerin and polyethylene glycol 400, and to 30% with water. It is generally regarded as relatively non-toxic and non-irritant at the concentrations used as a pharmaceutical excipient, but it can be irritant when used undiluted, its tolerability being approximately the same as that of propylene glycol.

**Formulation of the compositions:** A principal but not exclusive purpose of the invention is to provide the active ingredients of melatonin and/or antioxidant metabolite, derivative or analogue thereof in stable solutions at higher concentrations than are readily obtained with simple aqueous solutions. This is to enable high local concentrations of active ingredient to be reached when the solutions are topically applied to the skin or other epithelia, for example, to protect against the harmful effects of ionizing radiation. The compositions of the present invention will typically contain a concentration of the active ingredient or ingredients at a concentration in excess of 0.1% (w/w or w/v), such as any concentration in the range of 0.1% to 1% (w/w or w/v) of the composition, or even higher concentrations in the range of 1% to 5% w/w. Preferably, the active ingredient is present in the range of 0.1% to 5% (w/w or w/v), 0.5% to 4.5%, 1% to 4%, 1.5% to 3.5%, or 2% to 3%. If other active ingredients are added, such as a pharmaceutically acceptable form of vitamin E and/or coenzyme Q10 and/or alpha-lipoic acid and/or vitamin C, they can be added, for example, in an amount ranging from 25% to 200% by weight of the amount of melatonin or a metabolite, derivative or analogue thereof.

In one embodiment of the invention, the active ingredient or ingredients referred to above is/are dissolved in DMSO or a DMSO/water mixture, in which DMSO acts both as a solubilizer and as a preservative for the active ingredient. It is intended that DMSO shall be present in molar excess to the active ingredient to at least a 2-fold molar excess, a 3-fold molar excess, or any value of molar excess up to a 10-fold molar excess or even higher, such as any molar excess in the range of 10-fold to 2000-fold or more. The active ingredient may be dissolved initially in the DMSO, whereupon the desired amount of water is slowly added and mixed in, or the desired DMSO/water mixture may be prepared and the active ingredient dissolved in the mixture.

In another embodiment of the invention, the active ingredient or ingredients are dissolved in a mixture of water, one or more water-miscible, biologically compatible solubilizing agents of the many such agents known to the skilled person, and DMSO. The biocompatible solubilizing agent may, as a non-limiting example, be glycofurol. By the terms "biologically compatible" or "biocompatible" is meant compatibility with living tissue or a living system by not being toxic, injurious, or physiologically reactive and not causing immunological rejection. As for the first embodiment, the DMSO should be present in molar excess over the active ingredient, according to the range stated above.

Solutes can be added to the water in the above formulations. Such solutes may include hydrochloric acid, sodium hydroxide and biocompatible buffering agents, non-limiting examples being sodium dihydrogen phosphate and disodium hydrogen phosphate, sodium carbonate and bicarbonate, in order to adjust the pH. Tonicity-adjusting agents, such as for example sodium chloride or calcium chloride, may also be added, as well as a suitable preservative such as methyl and/or propyl parahydroxybenzoate. If the formulations are to be applied to the oral cavity, e.g. sublingually, sweeteners and or other flavoring substances may be added.

**Potential uses of the compositions:** The compositions of the invention can be used for topical application to the skin and other epithelia, including skin wounds or ulcers, the oropharyngeal cavity, the rectum, vagina, or the urothelium of the urinary bladder. The intention may be to provide a high local concentration of the active ingredient to protect the underlying epithelium and tissue form the damaging effects of ionizing radiation, or to act as an anti-inflammatory agent; but the local application can also be given as a carefully metered dose, e.g. as a sublingual spray, in order to achieve a systemic action of melatonin at a low dosage by bypassing the inefficient absorption and low bioavailability of melatonin when given as a conventional oral dosage to be swallowed into the stomach.

A further advantage of the invention is that DMSO aids the penetration of the active ingredient into the underlying cells, has a local anesthetic or analgesic effect, and has in itself free-radical scavenging and anti-inflammatory properties which may act additively or synergistically with the active ingredient melatonin and its metabolites, derivatives or analogues.

Accordingly, there is herein disclosed the use of DMSO as an additive in a solution comprising a solvent and at least one active ingredient selected from melatonin and/or an antioxidant metabolite, derivative or analogue thereof, for prolonging stability and/or shelf life of the at least one active ingredient in the solution by retarding a decomposition of the at least one active ingredient comprised in the solution. At least one active ingredient must be selected, which can be selected from melatonin and its various metabolites and derivatives as given above; however, the present invention does not exclude the presence of further active ingredients such as those other antioxidants mentioned above.

For such solutions comprising melatonin or a derivative as active ingredients, it has now surprisingly been observed, that when DMSO is added to a solution comprising the active ingredient, the active ingredient is present in said solution to at least 95% of initial amount after 45 days of storage at 25°C. Accordingly, DMSO arrests or hinders the degradation of melatonin and/or its derivatives and analogues as according to the above list very significantly. In experiments where DMSO was present, no measurable degradation was observed in HPLC following storage of such solutions at 25°C for 45 days.

In the invention and when the solution comprises DMSO and for compliance with the inventive concept underlying the suggested use, the active ingredient shall be present in the solution to at least 95% of initial amount after 45 days of storage at 25°C, to at least 97.5%, to at least 99%, to at least 99.5%, to at least 99.9%, or to at least 99.99%. Preferably, the active ingredient is present in the solution to at least 95% of initial amount after 30 days of storage at 25°C, preferably to at least 97.5%, to at least 99%, to at least 99.5%, to at least 99.9%, or even more preferably to at least 99.99%. More preferably, the active ingredient is present in the solution to at least 95% of initial amount after 40 days of storage at 25°C, preferably to at least 97.5%, to at least 99%, to at least 99.5%, to at least 99.9%, or even more preferably to at least 99.99%. And even more preferably, the active ingredient is present in the solution to at least 95% of initial amount after 45 days of storage at 25°C, preferably to at least 97.5%, to at least 99%, to at least 99.5%, to at least 99.9%, or even more preferably to at least 99.99%.

Further, there is disclosed with the present invention, the use of glycofurol as an additive in a solution comprising a solvent and at least one active ingredient selected from melatonin and/or an antioxidant metabolite, derivative or analogue thereof, for prolonging stability and/or shelf life of the at least one active ingredient in the solution by retarding a decomposition of the at least one active ingredient comprised in the solution. At least one active ingredient must be selected, which can be selected from melatonin and its various metabolites and derivatives as given above, however, the present invention does not exclude the presence of further active ingredients comprised in the above list.

For such solutions comprising melatonin or a derivative as active ingredients, it has now surprisingly been observed, that when glycofurol is added to a solution comprising the active ingredient, the active ingredient is present in said solution to at least 95% of initial amount after 45 days of storage at 25°C. Accordingly, glycofurol arrests or hinders the degradation of melatonin and/or its derivatives and analogues as according to the above list very significantly. In experiments where glycofurol was present, a degradation of less than 0.4% of initial was observed in HPLC following storage of such solutions at 25°C for 45 days.

In the invention and when the solution comprises glycofurol and for compliance with the inventive concept underlying the suggested use, the active ingredient shall be present in the solution to at least 95% of initial amount after 45 days of storage at 25°C, to at least 97.5%, to at least 99%, to at least 99.5%, or even more preferably to at least 99.7%. Preferably, the active ingredient is present in the solution to at least 95% of initial amount after 30 days of storage at 25°C, preferably to at least 97.5%, to at least 99%, to at least 99.5%, or even more preferably to at least 99.7%. More preferably, the active ingredient is present in the solution to at least 95% of initial amount after 40 days of storage at 25°C, preferably to at least 97.5%, to at least 99%, to at least 99.5%, or even more preferably to at least 99.7%. And even more preferably, the active ingredient is present in the solution to at least 95% of initial amount after 45 days of storage at 25°C, preferably to at least 97.5%, to at least 99%, to at least 99.5%, or even more preferably to at least 99.7%.

The determination of what constitutes initial amount and final amount shall be made using the same experimental techniques. In the experiments presented below, initial and final amounts of degradation products were determined using HPLC as an exemplary measurement technique. Standard melatonin solutions at fixed concentrations were prepared and within the sensitivity of the HPLC, no detectable initial degradation products could be determined. Accordingly, the purity of the tested samples was 100% of the initial calculated concentration. Hence this result serves the purpose of 100% initial amount, determining the scale of the experiment on which any later observed degradation is to be measured when using HPLC.

The skilled person will know that it cannot be ruled out; that using other detection techniques it may be determined that some degradation products are present initially, rendering the actual concentration lower than the calculated or intended concentration of melatonin and its derivatives. However, the actual concentration thus measured with this other detection technique will then serve the purpose of 100% against which, following the same other detection technique, degradation as a consequence of storage is to be measured.

It is intended, and generally preferred that the solvent used in dissolving melatonin and its derivatives, shall be water, or water comprising salts, preferably isotonic water. Further low-molecular weight organic solvents, such as e.g. ethanol, or glycols can alternatively be present to some extent, however it is contemplated and preferred that the main solvent matrix shall be water as detailed above.

It is preferred that when DMSO is present, it shall be present in a molar concentration at least 2-fold higher than the molar concentration of the at least one active ingredient, but preferably at least 5-fold higher, more preferably at least 10-fold higher, or even more preferably, at least 20-fold higher that the active ingredient or ingredients. In one embodiment, DMSO shall be present as an aqueous solution comprising between 20% (w/w) and 60% (w/w) of DMSO based on total mass of DMSO and water. In further embodiments, the solution comprises 40% (w/w) or 50% (w/w) of DMSO based on the total mass of DMSO and water.

In embodiments of the invention comprising glycofurol, the solutions obtained hereby are preferably an aqueous solution comprising between 10% (w/w) and 30% (w/w) of glycofurol based on the total mass of glycofurol and water; preferably 20% (w/w) of glycofurol.

Particularly suitable compositions arise when DMSO is added to a suitable, water-miscible and biocompatible solvent or solubilizer to obtain increased stability of melatonin and its derivatives. In such a solution comprising a solvent, at least one active ingredient selected from melatonin and/or an antioxidant metabolite, derivative or analogue thereof, and either DMSO, or a water-miscible and biocompatible solvent or solubilizer of the at least one active ingredient, or both DMSO and a water-miscible and biocompatible solvent or solubilizer of the at least one active ingredient, it has been observed that less DMSO is needed for stabilizing the melatonin solution. In such solutions it has been observed that the active ingredient is present in the solution to at least 95% of initial after 45 days of storage at 25°C, in fact to 100% after 45 days of storage at 45°C within the sensitivity of HPLC.

Glycofurol is such a suitable, water-miscible, biocompatible solvent or solubilizer for melatonin and its derivatives. In an embodiment of the solutions of the present invention comprising DMSO and glycofurol, the solution is an aqueous solution comprising between 10% (w/w) and 30% (w/w) of glycofurol based on the total mass of glycofurol and water; preferably 20% (w/w) of glycofurol. In another embodiment, the solution is an aqueous solution comprising 20% (w/w) of glycofurol based on the total mass of glycofurol and water; and 40% (w/w) of DMSO based on the total mass of DMSO and water.

The present invention accordingly further comprises such solutions as discussed above comprising melatonin or its derivatives and either DMSO, glycofurol, or DMSO and glycofurol in the amounts and concentrations detailed above, providing stability to melatonin and its derivatives according to the preferred embodiments for the uses of either DMSO, glycofurol, or DMSO and glycofurol in combination.

Further, there is herein disclosed compositions comprising the solutions of the invention as detailed above for topical application to a human subject for the treatment of a medical condition of the subject responsive to an active ingredient comprised in the composition, wherein the medical condition comprises a medical condition of the skin and other epithelia of the subject, such as a non-healing skin wound, an ulcer, an oropharyngeal cavity, the rectum, the vagina, or the urothelium of the urinary bladder; therein characterized by the active ingredient being present in the composition after 45 days of storage at 25°C to at least 95% of initial amount at preparation of the solution, but preferably above 99.5% after 45 days of storage at 25°C, in accordance with the respective degradation profiles and specific embodiment for respectively DMSO, glycofurol, or DMSO and glycofurol in combination.

Embodiments:
1. Use of dimethyl sulfoxide (DMSO) as an additive in a solution comprising a solvent and at least one active ingredient selected from melatonin and/or an antioxidant metabolite, derivative or analogue thereof, for prolonging the stability and/or shelf life of said at least one active ingredient in said solution by retarding a decomposition of said at least one active ingredient comprised in said solution.
2. A use of DMSO according to embodiment 1, wherein said active ingredient is present in said solution to at least 95% of initial amount after 45 days of storage at 25°C.
3. A use of DMSO according to either embodiment 1 or 2, wherein said solvent is water.
4. A use of DMSO according to any of embodiments 1 to 3, wherein said solution further comprises a water-miscible and biocompatible solvent or solubilizer of said at least one active ingredient.
5. A use of DMSO according to embodiment 4, wherein said water-miscible and biocompatible solvent or solubilizer is glycofurol.
6. A use of DMSO according to any of the preceding embodiments, wherein DMSO is present in a molar concentration at least 2-fold higher than the molar concentration of said at least one active ingredient.
7. A use of DMSO according to any of the preceding embodiments, wherein said solution is an aqueous solution comprising between 20% (w/w) and 60% (w/w) of DMSO based on the total mass of DMSO and water.
8. A use of DMSO according to any of the preceding embodiments, wherein said solution comprises 40% (w/w) or 50% (w/w) of DMSO based on the total mass of DMSO and water.
9. A use of DMSO according to any of the preceding embodiments, wherein said solution is an aqueous solution comprising between 10% (w/w) and 30% (w/w) of glycofurol based on the total mass of glycofurol and water, preferably 20% (w/w) of glycofurol.
10. A use of DMSO according to any of the preceding embodiments, wherein said solution is an aqueous solution comprising 20% (w/w) of glycofurol based on the total mass of glycofurol and water, and 40% (w/w) of DMSO based on the total amount of DMSO and water.
11. Use of glycofurol as an additive in a solution comprising a solvent and at least one active ingredient selected from melatonin and/or an antioxidant metabolite, derivative or analogue thereof, for prolonging stability and/or shelf life of said at least one active ingredient in said solution by retarding a decomposition of said at least one active ingredient comprised in said solution.
12. A use of glycofurol according to embodiment 11, wherein said active ingredient is present in said solution to at least 95% of initial amount after 45 days of storage at 25°C.
13. A use of glycofurol according to either embodiment 11 or 12, wherein said solvent is water.
14. A use of glycofurol according to any of the embodiments 11 to 13, wherein said solution is an aqueous solution comprising between 10% (w/w) and 30% (w/w) of glycofurol based on the total mass of glycofurol and water, preferably 20% (w/w) of glycofurol.
15. A use of glycofurol according to any of the embodiments 11 to 14, wherein said solution further comprises DMSO.
16. A use of glycofurol according to embodiment 15, wherein DMSO is present in a molar concentration at least 2-fold higher than the molar concentration of said at least one active ingredient.
17. A use of glycofurol according to either claim 15 or claim 16, wherein said solution is an aqueous solution comprising between 20% (w/w) and 60% (w/w) of DMSO based on the total mass of DMSO and water.
18. A use of glycofurol according to any of the embodiments 15 to 17, wherein said aqueous solution comprises 40% (w/w) or 50% (w/w) of DMSO based on the total mass of DMSO and water.
19. A use of glycofurol according to any of the embodiments 11 to 18, wherein said solution is an aqueous solution comprising 20% (w/w) of glycofurol based on the total mass of glycofurol and water, and 40% (w/w) of DMSO based on the total mass of DMSO and water.
20. A solution comprising a solvent, at least one active ingredient selected from melatonin and/or an antioxidant metabolite, derivative or analogue thereof, and either DMSO, or a water-miscible and biocompatible solvent or solubilizer of said at least one active ingredient, or both DMSO and a water-miscible and biocompatible solvent or solubilizer of said at least one active ingredient.
21. A solution according to embodiment 20, wherein said active ingredient is present in said solution to at least 95% of initial after 45 days of storage at 25°C.
22. A solution according to either embodiments 20 or 21, wherein said solvent is water.
23. A solution to any of the embodiments 20 to 22, wherein said water-miscible and biocompatible solvent or solubilizer of said at least one active ingredient is glycofurol.
24. A solution according to any of the embodiments 20 to 23, wherein DMSO, when present, is present in a molar concentration at least 2-fold higher than the molar concentration of said at least one active ingredient.
25. A solution according to any of the embodiments 20 to 24, wherein said solution is an aqueous solution comprising between 20% (w/w) and 60% (w/w) of DMSO based on the total mass of DMSO and water.
26. A solution according to embodiment 25, wherein said solution comprises 40% (w/w) or 50% (w/w) of DMSO based on the total mass of DMSO and water.
27. A solution according to any of the embodiments 20 to 26, wherein said solution is an aqueous solution comprising between 10% (w/w) and 30% (w/w) of glycofurol based on the total mass of glycofurol and water, preferably 20% (w/w) of glycofurol.
28. A solution according to any of the embodiments 20 to 27, wherein said solution is an aqueous solution comprising 20% (w/w) of glycofurol based on the total mass of glycofurol and water; and 40% (w/w) of DMSO based on the total mass of DMSO and water.
29. A composition comprising a solution according to any of the embodiments 20 to 28 for topical application to a human subject for treatment of a medical condition of said subject responsive to an active ingredient comprised in said composition, wherein said medical condition comprises a medical condition of the skin and other epithelia of said subject, such as a non-healing skin wound, an ulcer, the oropharyngeal cavity, the rectum, the vagina, or the urothelium of the urinary bladder; therein characterized by said active ingredient being present in said composition after 45 days of storage at 25°C to at least 95% of initial amount at preparation of said solution.
30. A composition according to embodiment 29, wherein the solution for topical application comprises between 1% (w/w) and 3% (w/w) of melatonin as the active ingredient in aqueous solution comprising between 10% (w/w) and 30% (w/w) of glycofurol, preferably 20% (w/w) of glycofurol, and between 20% (w/w) and 60% (w/w) of DMSO, preferably 40% (w/w) of DMSO, wherein the medical condition of the skin and other epithelia of the subject is a medical condition of the rectum, suchas radiation proctitis.
31. A composition according to embodiment 29, wherein the solution for topical application comprises between 1% (w/w) and 3% (w/w) of melatonin as the active ingredient in aqueous solution comprising between 10% (w/w) and 30% (w/w) of glycofurol, preferably 20% (w/w) of glycofurol, and between 20% (w/w) and 60% (w/w) of DMSO, preferably 40% (w/w) of DMSO, wherein the medical condition of the skin and other epithelia of the subject is a medical condition of the vagina, such as radiation vaginitis.
32. A composition according to embodiment 29, wherein the solution for topical application comprises between 0.05% (w/w) and 1% (w/w) of melatonin as the active ingredient in aqueous solution comprising between 20% (w/w) and 60% (w/w) of DMSO, preferably 50% (w/w) of DMSO, wherein the medical condition of the skin and other epithelia of the subject is a medical condition of the urothelium of the urinary bladder, such as radiation cystitis.
33. A composition according to embodiment 29, wherein the solution for topical application comprises between 1% (w/w) and 12.5% (w/w) of melatonin as the active ingredient in a cream or hydrogel in which the aqueous phase optionally comprises between 10% (w/w) and 30% (w/w) of glycofurol, preferably 20% (w/w) glycofurol, and comprises between 20% (w/w) and 60% (w/w) of DMSO, preferably 40% (w/w) of DMSO, wherein the medical condition of the skin and other epithelia of the subject is a medical condition of the skin, such as radiation dermatitis.
34. A method of preparing a solution comprising a solvent and at least one active ingredient selected from melatonin and/or an antioxidant metabolite derivative or analogue thereof, said method comprising dissolving an active ingredient selected from melatonin and/or an antioxidant metabolite, derivative or analogue thereof, in dimethyl sulfoxide (DMSO) and adding said solvent, wherein the stability and/or shelf life of said at least one active ingredient in said solution is prolonged by retarding a decomposition of said at least one active ingredient comprised in said solution.
35. A method according to embodiment 34, wherein said active ingredient is present in said solution to at least 95% of initial amount after 45 days of storage at 25°C.
36. A method according to embodiment 35, wherein said active ingredient is present in said solution to at least 95% of initial amount after 45 days of storage at 25°C.
37. A method according to either embodiment 35 or 36, wherein said solvent is water.
38. A method according to any of embodiments 35 to 37, wherein said solution further comprises a water-miscible and biocompatible solvent or solubilizer of said at least one active ingredient.
39. A method according to embodiment 38, wherein said water-miscible and biocompatible solvent or solubilizer is glycofurol.
40. A method according to any of the preceding embodiments, wherein DMSO is present in a molar concentration at least 2-fold higher than the molar concentration of said at least one active ingredient.
41. A method according to any of the preceding embodiments, wherein said solution is an aqueous solution comprising between 20% (w/w) and 60% (w/w) of DMSO based on the total mass of DMSO and water.
42. A method according to any of the preceding embodiments, wherein said solution comprises 40% (w/w) or 50% (w/w) of DMSO based on the total mass of DMSO and water.
43. A method according to any of the preceding embodiments, wherein said solution is an aqueous solution comprising between 10% (w/w) and 30% (w/w) of glycofurol based on the total mass of glycofurol and water, preferably 20% (w/w) of glycofurol.
44. A use of DMSO according to any of the preceding embodiments, wherein said solution is an aqueous solution comprising 20% (w/w) of glycofurol based on the total mass of glycofurol and water; and 40% (w/w) of DMSO based on the total mass of DMSO and water.

### Examples

### Inhibition by DMSO of melatonin decomposition in aqueous solutions stored at 25°C

The following non-limiting example illustrates the practice of the invention:
As a preliminary to the storage experiment to determine the stability of melatonin in an aqueous solution containing a water-miscible, biologically compatible solubilizer, the solubility of highly purified melatonin in 20% (w/w) aqueous propylene glycol and in 20% (w/w) aqueous glycofurol at 25°C was determined by overnight end-over-end rotation of an excess of finely powdered melatonin with samples of each of the mixtures. The samples were filtered through 0.45 µm pore-size Q-Max RR syringe filters and the concentration of melatonin in the filtrates was determined by highpressure liquid chromatography (HPLC) in a 100 x 4.6 mm Phenomenex Kinetex column of 5-µm particle-size XB-C18 silica eluted with a linear gradient of solvent B (acetonitrile containing 0.1% (v/v) acetic acid) 10% to 50% in solvent A (water containing 0.1% v/v acetic acid) from 0.8 to 7.8 min, returning to 10% B at 8.8 min. Peaks were detected by UV absorption at 277 µm wavelength. The mean concentration of melatonin in the 20% (w/w) aqueous propylene glycol samples was determined to be 3.7 mg/mL. This is within the range of solubility values for melatonin in 20% aqueous propylene glycol at 25°C reported by Lee (1990). In contrast, the solubility of melatonin in 20% (w/w) aqueous glycofurol was greater than 10 mg/mL (mean 10.8 mg/mL) under the same conditions. As it was desired to conduct stability studies with melatonin at a concentration of 10 mg/mL in a water/solubilizer mixture, the 20% (w/w) aqueous glycofurol was chosen as the medium for this study. To study the effect of adding DMSO to this medium, a medium consisting of 20% (w/w) glycofurol and 40% (w/w) DMSO in water was also prepared. In addition, the stability of melatonin at a concentration of 1 mg/mL in 50% w/w aqueous DMSO was studied in parallel.

The corresponding solutions were prepared, comprising 1) melatonin at 10 mg/mL in 20% (w/w) aqueous glycofurol; 2) melatonin at 10 mg/mL in 20% (w/w) glycofurol and 40% (w/w) DMSO in water; and 3) melatonin at 10 mg/mL in 50% (w/w) aqueous DMSO. In solution 2), DMSO was calculated to be present in approximately 119-fold molar excess over melatonin; in solution 3), DMSO was calculated to be in approximately 1487-fold molar excess over melatonin. Aliquots of each of the three solutions in hermetically sealed tubes were stored at 25°C for a total of 45 days, the melatonin concentrations being determined by HPLC on day 0, and in triplicate HPLC determinations on days 10, 17, 31 and 45. Over this period, no significant decline in the melatonin concentrations was observed. It was concluded that melatonin is considerably more stable in all of these solutions than in 20% (w/w) aqueous propylene glycol, for which Lee (1990) reported a decline to 73.54% or less in the measured melatonin concentration after 7 days of storage at 25°C.

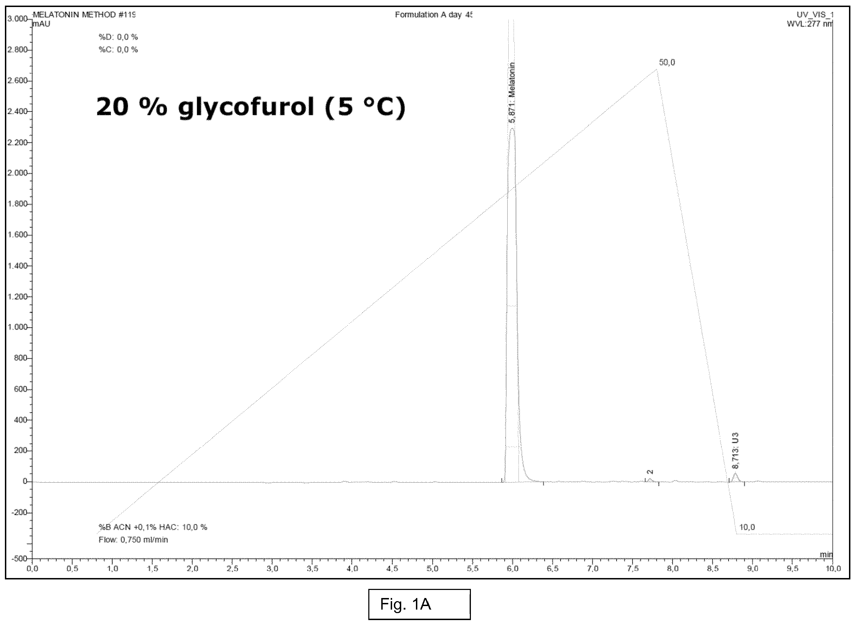

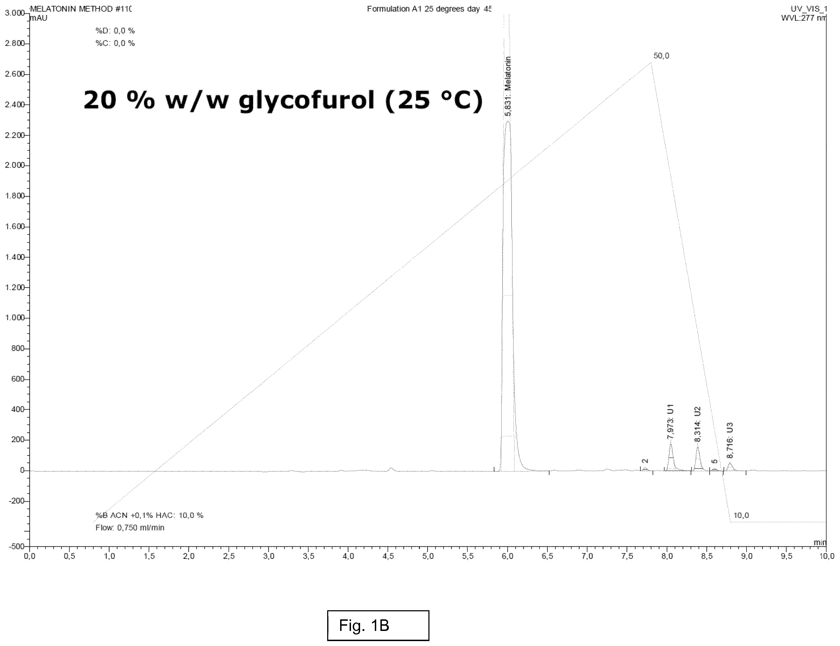

Figure 1 presents results detailing and illustrating the invention. High pressure liquid chromatography (HPLC) elution profiles obtained from a 100 x 4.6 mm Phenomenex Kinetex column of XB-C18 silica (5 µm particle size) eluted with a linear gradient of solvent B (acetonitrile containing 0.1% v/v acetic acid) 10% to 50% in solvent A (water containing 0.1% v/v acetic acid) from 0.8 to 7.8 min, returning to 10% B at 8.8 min. Peaks were detected by UV absorption at 277 µm wavelength. Overloading sample sizes were used to facilitate the detection of small amounts of breakdown products. Samples: Melatonin 10 mg/mL in 20% (w/w) aqueous glycofurol, stored for 45 days at 5°C (Figure 1A) or 25°C (Figure 1B). Melatonin emerges at 6.0 min, measured as 5871 absorption units for the 5°C sample and 5831 absorption units for the 25°C sample. Small invariant peaks of 2 and 8.7 absorption units at 7.7 and 8.8 min, respectively, were seen in all samples containing 20% (w/w) glycofurol and were attributed to trace impurities in this solvent. The 25°C sample contained peaks of 7.973, 8.314 and 5 absorption units at 8.1, 8.4 and 8.6 min, respectively, which were not seen in the 5°C sample.

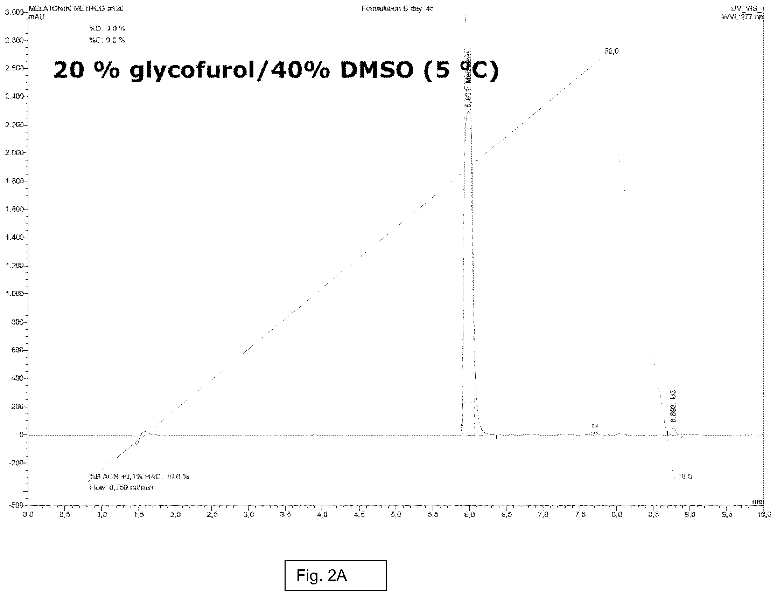

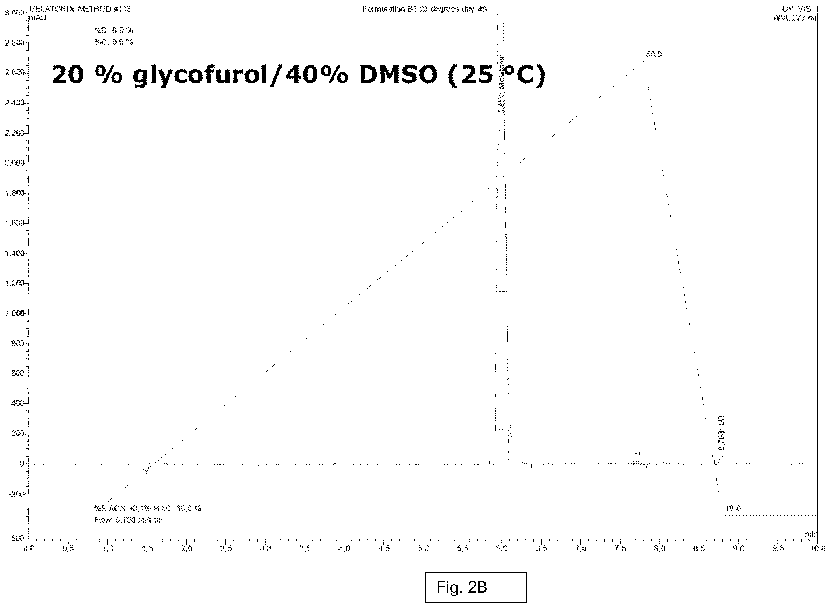

Figure 2 presents further results detailing and illustrating the invention. HPLC elution profiles were obtained as described for Figure 1. Samples: Melatonin 10 mg/mL in 20% (w/w) glycofurol, 40% (w/w) DMSO in water stored for 45 days at 5°C (Figure 2A) or 25°C (Figure 2B). The melatonin peak emerging at 6.0 min was measured as 6831 absorption units for the 5°C sample and 6851 absorption units for the 25°C sample. Small invariant peaks of 2 and 8.7 absorption units at 7.7 and 8.8 min, respectively, were seen in all samples containing 20% (w/w) glycofurol and were attributed to trace impurities in this solvent. The 25°C sample contained no additional peaks of UV-absorbent material.

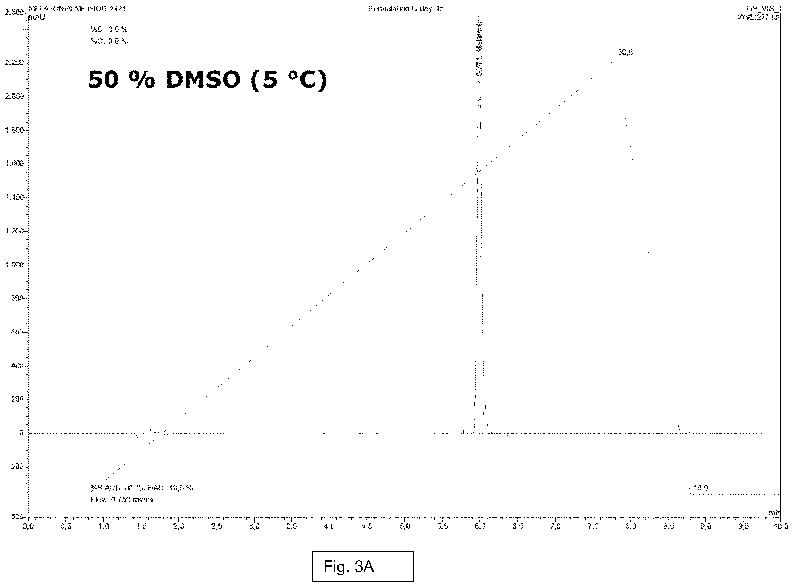

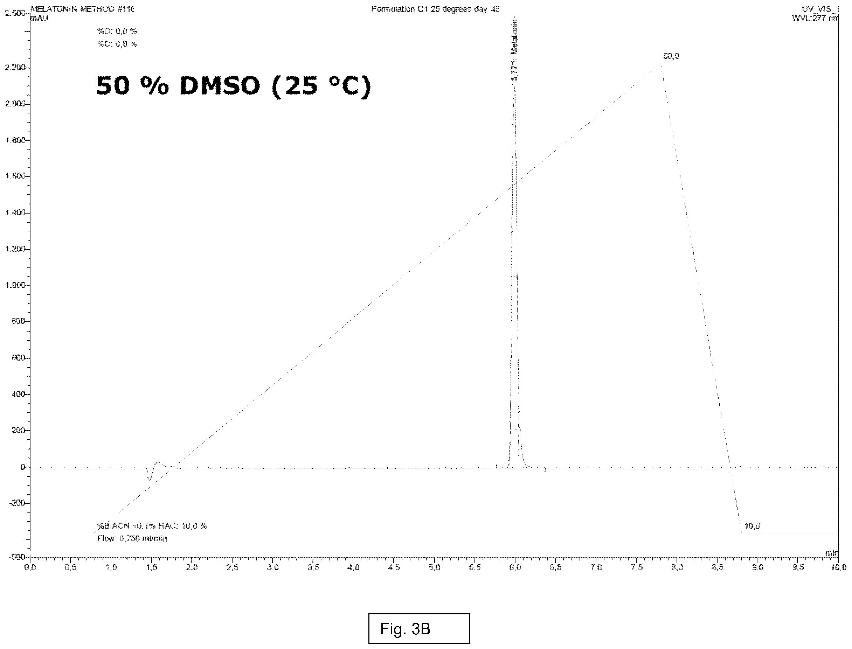

Figure 3 presents further results detailing and illustrating the invention. HPLC elution profiles were obtained as described for Figure 1. Samples: Melatonin 10 mg/mL in 50% (w/w) aqueous DMSO stored for 45 days at 5°C (Figure 3A) or 25°C (Figure 3B). The melatonin peak emerging at 6.0 min was measured as 8771 absorption units for both the 5°C and 25°C samples. No additional peaks were detected.

Figures 1, 2 and 3 show the results of an experiment to detect the possible breakdown of melatonin by determining the HPLC elution profiles for each of the three solutions of melatonin stored for 45 days at 25°C as compared with those for the same solutions stored for 45 days at 5°C. In the 20% (w/w) aqueous glycofurol solution, a small degree of melatonin degradation at 25°C was detected by the appearance of very small peaks of UV-absorbent material at 8.1, 8.4 and 8.6 min, respectively, which were not seen in the sample stored at 5°C. Together, these peaks amounted to 0.36% of the recovered UV-absorbent material (Figure 1). These peaks were not seen in the equivalent sample of the solution in 20% (w/w) glycofurol, 40% (w/w) DMSO in water (Figure 2), nor in the solution in 50% (w/w) DMSO (Figure 3). In the two latter solutions there was no evidence of the melatonin breakdown after 45 days of storage at 25°C.

From the experiments it can be gathered that both glycofurol and DMSO are effective retardants for the decomposition of melatonin, even after 45 days of storage at 25°C. Glycofurol appears more effective per mole of added glycofurol to the solution comprising melatonin than DMSO at retarding the decomposition of melatonin although at the tested concentrations of glycofurol a slightly higher decomposition rate of melatonin was observed. However, as shown in the experiments this slightly higher decomposition rate could be completely compensated for by the addition of DMSO as a further additive, wherein both additives (glycofurol and DMSO) provide an additive and supplementary effect on the stability of melatonin and its derivatives.

From the data it can be concluded that the addition of DMSO or glycofurol to solutions and compositions comprising melatonin and/or its derivatives provide a stabilizing effect on these compositions against decomposition for at least 45 days at 25°C, making such solutions and compositions highly suitable for the intended uses herein and for solving the technical problems as detailed by the present inventors herein.

### References

Cavallo A, Hassan M (1995) Stability of melatonin in aqueous solution. J Pineal Res 18:90-92.
Daya S, Walker RB, Glass BD, Anoopkumar-Dukie S (2001). The effect of variations in pH and temperature on stability of melatonin in aqueous solution. J. Pineal Res 31:155-158.
EC SCCS/1315/10 (2010) European Commission Directorate-General for Health and Consumers, Scientific Committee on Consumer Safety, SCCS, Opinion on Melatonin. SCCS/1315/10, 24.3.10.
Lee Y-L (1990) Development of a transdermal delivery system for melatonin. MSc thesis submitted to Oregon State University.
Maharaj DS, Glass BD, Daya S (2007) Melatonin: new places in therapy. Biosci Rep 27:299-320.
Oxenkrug G (2005) Antioxidant effects of N-acetylserotonin: possible mechanisms and clinical implications. Ann N Y Acad Sci 1053:334-347.
Reiter RJ, Tan DX, Terron MP, Flores LJ, Czarnocki Z (2007) Melatonin and its metabolites: new findings regarding their production and their radical scavenging actions. Acta Biochim Pol 54:1-9.
Shirley SW, Stewart BH, Mirelman S (1978) Dimethyl sulfoxide in treatment of inflammatory genitourinary disorders. Urology 11:215-220.
Tan DX, Chen LD, Poeggeler B, Manchester LC, Reiter RJ (1993) Melatonin: a potent, endogenous hydroxyl radical scavenger. Endocrine J 1:57-60.
Vijayalaxmi, Reiter RJ, Tan DX, Herman TS, Thomas CR Jr (2004) Melatonin as a radioprotective agent: a review. Int J Radiat Oncol Biol Phys 59:639-653.

## Claims

1. A composition comprising a solvent, at least one active ingredient being melatonin, and either DMSO, or both DMSO and a water-miscible and biocompatible solvent or solubilizer of said at least one active ingredient for use in a topical application to a human subject in treatment of a medical condition of said subject responsive to an active ingredient comprised in said composition, wherein said medical condition comprises a medical condition of the skin and other epithelia of said subject, such as a non-healing skin wound, an ulcer, the oropharyngeal cavity, the rectum, the vagina, or the urothelium of the urinary bladder; **characterized by** said active ingredient being present in said composition after 45 days of storage at 25°C to at least 95% of initial amount at preparation of said solution.

2. The composition comprising a solvent, at least one active ingredient being melatonin, and either DMSO, or both DMSO and a water-miscible and biocompatible solvent or solubilizer of said at least one active ingredient for use in the topical application as defined in claim 1, wherein said solvent is water.

3. The composition comprising a solvent, at least one active ingredient selected from melatonin, and either DMSO, or both DMSO and a water-miscible and biocompatible solvent or solubilizer of said at least one active ingredient for use in the topical application as defined in any of the claims 1 or 2, wherein said water-miscible and biocompatible solvent or solubilizer of said at least one active ingredient is glycofurol.

4. The composition comprising a solvent, at least one active ingredient being melatonin, and either DMSO, or both DMSO and a water-miscible and biocompatible solvent or solubilizer of said at least one active ingredient for use in a topical application as defined in any of the claims 1-3, wherein DMSO is present in a molar concentration at least 2-fold higher than the molar concentration of said at least one active ingredient.

5. The composition comprising a solvent, at least one active ingredient being melatonin, and either DMSO, or both DMSO and a water-miscible and biocompatible solvent or solubilizer of said at least one active ingredient for use in a topical application as defined in any of the claims 1-4, wherein said solution is an aqueous solution comprising between 20% (w/w) and 60% (w/w) of DMSO based on the total mass of DMSO and water.

6. The composition comprising a solvent, at least one active ingredient being melatonin, and either DMSO, or both DMSO and a water-miscible and biocompatible solvent or solubilizer of said at least one active ingredient for use in a topical application as defined in any of the claims 1-5, wherein said solution comprises 40% (w/w) or 50% (w/w) of DMSO based on the total mass of DMSO and water.

7. The composition comprising a solvent, at least one active ingredient being melatonin, and either DMSO, or both DMSO and a water-miscible and biocompatible solvent or solubilizer of said at least one active ingredient for use in a topical application as defined in any of the claims 1-6, wherein said solution is an aqueous solution comprising between 10% (w/w) and 30% (w/w) of glycofurol based on the total mass of glycofurol and water, preferably 20% (w/w) of glycofurol.

8. The composition comprising a solution comprising a solvent, at least one active ingredient being melatonin, and either DMSO, or both DMSO and a water-miscible and biocompatible solvent or solubilizer of said at least one active ingredient for use in a topical application as defined in any of the claims 1-7, wherein said solution is an aqueous solution comprising 20% (w/w) of glycofurol based on the total mass of glycofurol and water, and 40% (w/w) of DMSO based on the total mass of DMSO and water.

9. The composition comprising a solvent, at least one active ingredient being melatonin, and either DMSO, or both DMSO and a water-miscible and biocompatible solvent or solubilizer of said at least one active ingredient for use in a topical application as defined in any of the claims 1-7, wherein the solution comprises between 1% (w/w) and 3% (w/w) of melatonin as the active ingredient in aqueous solution comprising 20% (w/w) of glycofurol, and 40% (w/w) of DMSO and wherein the medical condition of the skin and other epithelia of the subject is a medical condition of the rectum, such as radiation proctitis.

10. The composition comprising a solvent, at least one active ingredient being melatonin, and either DMSO, or both DMSO and a water-miscible and biocompatible solvent or solubilizer of said at least one active ingredient for use in a topical application as defined in any of the claims 1-7, wherein the solution comprises between 1% (w/w) and 3% (w/w) of melatonin as the active ingredient in aqueous solution comprising between 10% (w/w) and 30% (w/w) of glycofurol, preferably 20% (w/w) of glycofurol, and between 20% (w/w) and 60% (w/w) of DMSO, preferably 40% (w/w) of DMSO, wherein the medical condition of the skin and other epithelia of the subject is a medical condition of the vagina, such as radiation vaginitis.

11. The composition comprising a solvent, at least one active ingredient being melatonin, and either DMSO, or both DMSO and a water-miscible and biocompatible solvent or solubilizer of said at least one active ingredient for use in a topical application as defined any of the claims 1-7, wherein the solution comprises between 0.05% (w/w) and 1% (w/w) of melatonin as the active ingredient in aqueous solution comprising between 20% (w/w) and 60% (w/w) of DMSO, preferably 50% (w/w) of DMSO, wherein the medical condition of the skin and other epithelia of the subject is a medical condition in the urothelium of the urinary bladder, such as radiation cystitis.

12. The composition comprising a solvent, at least one active ingredient being melatonin, and either DMSO, or both DMSO and a water-miscible and biocompatible solvent or solubilizer of said at least one active ingredient for use in a topical application as defined in any of the claims 1-7, wherein the solution comprises between 1% (w/w) and 12.5% (w/w) of melatonin as the active ingredient in a cream or hydrogel in which the aqueous phase optionally comprises between 10% (w/w) and 30% (w/w) of glycofurol, preferably 20% (w/w) glycofurol, and comprises between 20% (w/w) and 60% (w/w) of DMSO, preferably 40% (w/w) of DMSO, wherein the medical condition of the skin and other epithelia of the human is a medical condition of the skin, such as radiation dermatitis.

## Patentansprüche

1. Zusammensetzung umfassend ein Lösungsmittel, wobei mindestens ein Wirkstoff Melatonin und entweder DMSO oder sowohl DMSO als auch ein Wasser-mischbares und biokompatibles Lösungsmittel oder Lösungsvermittler des mindestens einen Wirkstoffs ist zur Verwendung in einer topischen Anwendung für ein menschliches Individuum bei der Behandlung eines medizinischen Zustands des Individuums, das auf einen Wirkstoff reagiert, der in der Zusammensetzung enthalten ist, wobei der medizinische Zustand einen medizinischen Zustand der Haut und anderer Epithele des Individuums, wie beispielsweise einer nicht abheilenden Hautwunde, eines Geschwürs, des Oropharyngealhohlraums, des Enddarms, der Scheide oder des Urothels der Harnblase, umfasst; **dadurch gekennzeichnet, dass** der Wirkstoff in der Zusammensetzung nach 45 Tagen Aufbewahrung bei 25° C bis zu mindestens 95% der anfänglichen Menge bei der Herstellung der Lösung vorhanden ist.

2. Zusammensetzung umfassend ein Lösungsmittel, wobei mindestens ein Wirkstoff Melatonin und entweder DMSO oder sowohl DMSO als auch ein Wasser-mischbares und biokompatibles Lösungsmittel oder Lösungsvermittler des mindestens einen Wirkstoffs ist zur Verwendung in der topischen Anwendung nach Anspruch 1, wobei das Lösungsmittel Wasser ist.

3. Zusammensetzung umfassend ein Lösungsmittel, wobei mindestens ein Wirkstoff aus Melatonin und entweder DMSO oder sowohl DMSO als auch einem Wasser-mischbaren und biokompatiblen Lösungsmittel oder Lösungsvermittler des mindestens einen Wirkstoffs ausgewählt ist zur Verwendung in der topischen Anwendung nach einem der Ansprüche 1 oder 2, wobei das Wasser-mischbare und biokompatible Lösungsmittel oder der Lösungsvermittler des mindestens einen Wirkstoffs Glycofurol ist.

4. Zusammensetzung umfassend ein Lösungsmittel, wobei mindestens ein Wirkstoff Melatonin und entweder DMSO oder sowohl DMSO als auch ein Wasser-mischbares und biokompatibles Lösungsmittel oder Lösungsvermittler des mindestens einen Wirkstoffs ist zur Verwendung in einer topischen Anwendung nach einem der Ansprüche 1-3, wobei DMSO in einer Molkonzentration von mindestens zweimal höher als die Molkonzentration des mindestens einen Wirkstoffs vorhanden ist.

5. Zusammensetzung umfassend ein Lösungsmittel, wobei mindestens ein Wirkstoff Melatonin und entweder DMSO oder sowohl DMSO als auch ein Wasser-mischbares und biokompatibles Lösungsmittel oder Lösungsvermittler des mindestens einen Wirkstoffs ist zur Verwendung in einer topischen Anwendung nach einem der Ansprüche 1-4, wobei die Lösung eine wässrige Lösung ist umfassend zwischen 20% (Gew./Gew.) und 60% (Gew./Gew.) von DMSO basierend auf der Gesamtmasse von DMSO und Wasser.

6. Zusammensetzung umfassend ein Lösungsmittel, wobei mindestens ein Wirkstoff Melatonin und entweder DMSO oder sowohl DMSO als auch ein Wasser-mischbares und biokompatibles Lösungsmittel oder Lösungsvermittler des mindestens einen Wirkstoffs ist zur Verwendung in einer topischen Anwendung nach einem der Ansprüche 1-5, wobei die Lösung 40% (Gew./Gew.) oder 50% (Gew./Gew.) von DMSO basierend auf der Gesamtmasse von DMSO und Wasser umfasst.

7. Zusammensetzung umfassend ein Lösungsmittel, wobei mindestens ein Wirkstoff Melatonin und entweder DMSO oder sowohl DMSO als auch ein Wasser-mischbares und biokompatibles Lösungsmittel oder Lösungsvermittler des mindestens einen Wirkstoffs ist zur Verwendung in einer topischen Anwendung nach einem der Ansprüche 1-6, wobei die Lösung eine wässrige Lösung ist umfassend zwischen 10% (Gew./Gew.) und 30% (Gew./Gew.) von Glycofurol basierend auf der Gesamtmasse von Glycofurol und Wasser, vorzugsweise 20% (Gew./Gew.) von Glycofurol.

8. Zusammensetzung umfassend eine Lösung umfassend ein Lösungsmittel, wobei mindestens ein Wirkstoff Melatonin und entweder DMSO oder sowohl DMSO als auch ein Wasser-mischbares und biokompatibles Lösungsmittel oder Lösungsvermittler des mindestens einen Wirkstoffs ist zur Verwendung in einer topischen Anwendung nach einem der Ansprüche 1-7, wobei die Lösung eine wässrige Lösung ist umfassend 20% (Gew./Gew.) von Glycofurol basierend auf der Gesamtmasse von Glycofurol und Wasser, und 40% (Gew./Gew.) von DMSO basierend auf der Gesamtmasse von DMSO und Wasser.

9. Zusammensetzung umfassend ein Lösungsmittel, wobei mindestens ein Wirkstoff Melatonin und entweder DMSO oder sowohl DMSO als auch ein Wasser-mischbares und biokompatibles Lösungsmittel oder Lösungsvermittler des mindestens einen Wirkstoffs ist zur Verwendung in einer topischen Anwendung nach einem der Ansprüche 1-7, wobei die Lösung zwischen 1% (Gew./Gew.) und 3% (Gew./Gew.) von Melatonin als der Wirkstoff in wässriger Lösung umfassend 20% (Gew./Gew.) von Glycofurol, und 40% (Gew./Gew.) von DMSO umfasst, und wobei der medizinische Zustand der Haut und anderer Epithele des Individuums ein medizinischer Zustand des Enddarms ist, wie beispielsweise Strahlungsproktitis.

10. Zusammensetzung umfassend ein Lösungsmittel, wobei mindestens ein Wirkstoff Melatonin und entweder DMSO oder sowohl DMSO als auch ein Wasser-mischbares und biokompatibles Lösungsmittel oder Lösungsvermittler des mindestens einen Wirkstoffs ist zur Verwendung in einer topischen Anwendung nach einem der Ansprüche 1-7, wobei die Lösung zwischen 1% (Gew./Gew.) und 3% (Gew./Gew.) von Melatonin als der Wirkstoff in wässriger Lösung umfassend zwischen 10% (Gew./Gew.) und 30% (Gew./Gew.) von Glycofurol, vorzugsweise 20% (Gew./Gew.) von Glycofurol, und zwischen 20% (Gew./Gew.) und 60% (Gew./Gew.) von DMSO, vorzugsweise 40% (Gew./Gew.) von DMSO umfasst, wobei der medizinische Zustand der Haut und anderer Epithele des Individuums ein medizinischer Zustand der Scheide ist, wie Strahlungsvaginitis.

11. Zusammensetzung umfassend ein Lösungsmittel, wobei mindestens ein Wirkstoff Melatonin und entweder DMSO oder sowohl DMSO als auch ein Wasser-mischbares und biokompatibles Lösungsmittel oder Lösungsvermittler des mindestens einen Wirkstoffs ist zur Verwendung in einer topischen Anwendung nach einem der Ansprüche 1-7, wobei die Lösung zwischen 0,05% (Gew./Gew.) und 1% (Gew./Gew.) von Melatonin als der Wirkstoff in wässriger Lösung umfassend zwischen 20% (Gew./Gew.) und 60% (Gew./Gew.) von DMSO, vorzugsweise 50% (Gew./Gew.) von DMSO umfasst, wobei der medizinische Zustand der Haut und anderer Epithele des Individuums ein medizinischer Zustand im Urothel der Harnblase ist, wie Strahlungscystitis.

12. Zusammensetzung umfassend ein Lösungsmittel, wobei mindestens ein Wirkstoff Melatonin und entweder DMSO oder sowohl DMSO als auch ein Wasser-mischbares und biokompatibles Lösungsmittel oder Lösungsvermittler des mindestens einen Wirkstoffs ist zur Verwendung in einer topischen Anwendung nach einem der Ansprüche 1-7, wobei die Lösung zwischen 1% (Gew./Gew.) und 12,5% (Gew./Gew.) von Melatonin als der Wirkstoff in einer Creme oder einem Hydrogel, worin die wässrige Phase eventuell zwischen 10% (Gew./Gew.) und 30% (Gew./Gew.) von Glycofurol, vorzugsweise 20% (Gew./Gew.) Glycofurol umfasst, und zwischen 20% (Gew./Gew.) und 60% (Gew./Gew.) von DMSO, vorzugsweise 40% (Gew./Gew.) von DMSO umfasst, wobei der medizinische Zustand der Haut und anderer Epithele des Menschen ein medizinischer Zustand der Haut ist, wie Strahlungsdermatitis.

## Revendications

1. Composition comprenant un solvant, au moins un principe actif étant de la mélatonine, et soit du DMSO, soit à la fois du DMSO et un solvant ou un solubilisant miscible dans l'eau et biocompatible dudit au moins un principe actif destiné à être utilisé dans une application topique à un sujet humain dans le traitement d'un état médical dudit sujet en réponse à un principe actif compris dans ladite composition, ledit état médical comprenant un état médical de la peau et d'autres épithéliums dudit sujet, tel qu'une plaie cutanée non cicatrisante, un ulcère, la cavité oropharyngée, le rectum, le vagin, ou l'urothélium de la vessie urinaire ; **caractérisé en ce que** ledit ingrédient actif est présent dans ladite composition après 45 jours de stockage à 25°C à au moins 95% de la quantité initiale lors de la préparation de ladite solution.

2. Composition comprenant un solvant, au moins un principe actif étant de la mélatonine, et soit du DMSO, soit à la fois du DMSO et un solvant ou un solubilisant miscible à l'eau et biocompatible dudit au moins un principe actif destiné à être utilisé dans l'application topique telle que définie dans la revendication 1, ledit solvant étant de l'eau.

3. Composition comprenant un solvant, au moins un principe actif choisi parmi la mélatonine, et soit le DMSO, soit le DMSO et un solvant ou un solubilisant miscible dans l'eau et biocompatible dudit au moins un principe actif pour une utilisation dans l'application topique telle que définie dans l'une quelconque des revendications 1 ou 2, dans laquelle ledit solvant ou solubilisant miscible à l'eau et biocompatible dudit au moins un principe actif est le glycofurol.

4. Composition comprenant un solvant, au moins un principe actif étant de la mélatonine, et soit du DMSO, soit à la fois du DMSO et un solvant ou un solubilisant miscible dans l'eau et biocompatible dudit au moins un principe actif pour une utilisation dans une application topique telle que définie dans l'une quelconque des revendications 1 à 3, dans laquelle le DMSO est présent dans une concentration molaire au moins 2 fois supérieure à la concentration molaire dudit au moins un principe actif.

5. Composition comprenant un solvant, au moins un principe actif étant de la mélatonine, et soit du DMSO, soit à la fois du DMSO et un solvant ou un solubilisant miscible à l'eau et biocompatible dudit au moins un principe actif destiné à être utilisé dans une application topique telle que définie dans l'une quelconque des revendications 1 à 4, dans laquelle ladite solution est une solution aqueuse comprenant entre 20% (p/p) et 60% (p/p) de DMSO sur la base de la masse totale de DMSO et d'eau.

6. Composition comprenant un solvant, au moins un principe actif étant de la mélatonine, et soit du DMSO, soit à la fois du DMSO et un solvant ou un solubilisant miscible à l'eau et biocompatible dudit au moins un principe actif destiné à être utilisé dans une application topique telle que définie dans l'une quelconque des revendications 1 à 5, dans laquelle ladite solution comprend 40% (p/p) ou 50% (p/p) de DMSO sur la base de la masse totale de DMSO et d'eau.

7. Composition comprenant un solvant, au moins un principe actif étant de la mélatonine, et soit du DMSO, soit à la fois du DMSO et un solvant ou un solubilisant miscible à l'eau et biocompatible dudit au moins un principe actif destiné à être utilisé dans une application topique telle que définie dans l'une quelconque des revendications 1 à 6, dans laquelle ladite solution est une solution aqueuse comprenant entre 10% (p/p) et 30% (p/p) de glycofurol sur la base de la masse totale de glycofurol et d'eau, de préférence 20% (p/p) de glycofurol.

8. Composition comprenant un solution, au moins un solvant, au moins un principe actif étant de la mélatonine, et soit du DMSO, soit à la fois du DMSO et un solvant ou un solubilisant miscible à l'eau et biocompatible dudit au moins un principe actif destiné à être utilisé dans une application topique telle que définie dans l'une quelconque des revendications 1 à 7, dans laquelle ladite solution est une solution aqueuse comprenant 20% (p/p) de glycofurol sur la base de la masse totale de glycofurol et d'eau, et 40% (p/p) de DMSO sur la base de la masse totale de DMSO et d'eau

9. Composition comprenant un solvant, au moins un principe actif étant de la mélatonine, et soit du DMSO, soit à la fois du DMSO et un solvant ou un solubilisant miscible à l'eau et biocompatible dudit au moins un principe actif destiné à être utilisé dans l'application topique telle que définie dans l'une quelconque des revendications 1 à 7, dans laquelle la solution comprend entre 1% (p/p) et 3% (p/p) de mélatonine en tant que principe actif dans une solution aqueuse comprenant 20% (p/p) de glycofurol et 40% (p/p) de DMSO, et dans laquelle l'état médical de la peau et d'autres épithelia du sujet est un état médical du rectum, tel qu'une provision de rayonnement.

10. Composition comprenant un solvant, au moins un principe actif étant de la mélatonine, et soit du DMSO, soit à la fois du DMSO et un solvant ou un solubilisant miscible à l'eau et biocompatible dudit au moins un principe actif destiné à être utilisé dans une application topique telle que définie dans l'une quelconque des revendications 1 à 7, dans laquelle la solution comprend entre 1% (p/p) et 3% (p/p) de mélatonine en tant que principe actif dans une solution aqueuse comprenant entre 10% (p/p) et 30% (p/p) de glycofurol, de préférence 20% (p/p) de glycofurol, et entre 20% (p/p) et 60% (p/p) de DMSO, de préférence 40% (p/p) de DMSO, l'état médical de la peau et d'autres épithelia du sujet étant un état médical du vagin, tel qu'une vaginite de rayonnement.

11. Composition comprenant un solvant, au moins un principe actif étant de la mélatonine, et soit du DMSO, soit à la fois du DMSO et un solvant ou un solubilisant miscible à l'eau et biocompatible dudit au moins un principe actif destiné à être utilisé dans une application topique telle que définie dans l'une quelconque des revendications 1 à 7, dans laquelle la solution comprend entre 0,05% (p/p) et 1% (p/p) de mélatonine en tant que principe actif dans une solution aqueuse comprenant entre 20% (p/p) et 60% (p/p) de DMSO, de préférence 50% (p/p) de DMSO, l'état médical de la peau et d'autres épithelia du sujet étant un état médical dans l'urothélium de la vessie urinaire, tel qu'une cystite de rayonnement.

12. Composition comprenant un solvant, au moins un principe actif étant de la mélatonine, et soit du DMSO, soit à la fois du DMSO et un solvant ou un solubilisant miscible à l'eau et biocompatible dudit au moins un principe actif destiné à être utilisé dans une application topique telle que définie dans l'une quelconque des revendications 1 à 7, dans laquelle la solution comprend entre 1% (p/p) et 12,5% (p/p) de mélatonine en tant que principe actif dans une crème ou un hydrogel dans lequel la phase aqueuse comprend éventuellement entre 10% (p/p) et 30% (p/p) de glycofurol, de préférence 20% (p/p) de glycofurol, et comprend entre 20% (p/p) et 60% (p/p) de DMSO, de préférence 40% (p/p) de DMSO, l'état médical de la peau et d'autres épithelia de l'être humain étant un état médical de la peau, tel qu'une dermatite de rayonnement.
